# EUROPEAN PATENT APPLICATION

(11) **EP 1 652 854 A1**
(43) Date of publication of application: **03.05.2006**
(21) Application number: 04771003.3
(22) Date of filing: 21.07.2004
(51) Int. Cl.: C07H 19/23, A61K 31/7056, A61P 35/00

(54) **INDOLOPYRROLOCABAZOLE DERIVATIVE AND ANTITUMOR AGENT**

(30) Priority: 24.07.2003 JP 2003009392
(71) Applicant: BANYU PHARMACEUTICAL CO., LTD., Chuo-ku, Tokyo 103-8416 (JP)
(72) Inventor: OHKUBO, Mitsuru, BANYU PHARMACEUTICAL CO., LTD., Tsukuba-shi, Ibaraki 3002611 (JP); ARAKAWA, Hiroharu, BANYU PHARMACEUTICAL CO., LTD., Tsukuba-shi, Ibaraki 3002611 (JP)
(74) Representative: Rollins, Anthony John
(86) International application number: PCT/JP2004/010742
(87) International publication number: WO 2005/010020

(57) **Abstract**

The present invention relates to new indolopyrrolocarbazole derivatives of formula (I): 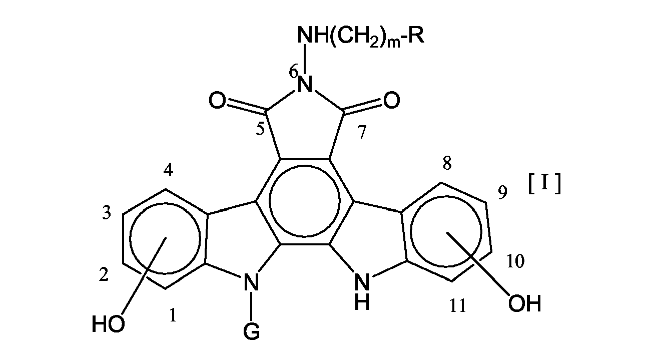 wherein R represents an unsubstitued pyridyl, furyl, or thienyl group; m represents an integer of 1 to 3; and G represents a β-D-glucopyranosyl group; and the positions of substitution of the hydroxyl groups on the indolopyrrolocarbazole ring are the 1- and 11-positions, or the 2- and 10-positions.

## Description

### Technical Field

The present invention relates to novel indolopyrrolocarbazole derivatives and use thereof as anti-tumor agents, which inhibit the growth of tumor cells to exhibit anti-tumor effects and are useful in the medicinal field.

### Background Art

A large number of compounds have already been used practically as pharmaceuticals in the field of cancer chemotherapy. The effect against a variety of tumors, however, is not always satisfactory, and additionally a problem of resistance of tumor cells to the drugs has complicated the clinical use [see the 47th Conference of Japanese Cancer Association, Report, pages 12-15 (1988)].

Under such situation, it has always been desired that a new anti-cancer substance is developed in the field of cancer therapy. Particularly, it is necessitated to overcome the resistance in the existing anti-cancer substances and to provide a substance effective to some of cancer against which the existing anti-cancer substances cannot show satisfactory effects.

In view of the foregoing, the present inventors have screened a wide variety of microbial metabolites and, as a result, they have found a novel anti-tumor compound BE-13793C (12,13-dihydrol-11-dihydroxy-5H-indolo[2,3-a]pyrrolo[3,4-c]carbazole-5,7(6H)-dione) (see European Patent Publication No.0388956A2).

Thereafter, the present inventors have attempted to create a more potent anti-tumor compound by chemical modification of BE-13793C and disclosed the result in the prior applications and patents (European Patent Publication No. 0528030A1, USP 5,591,842, USP 5,668,271, USP 5,804,564, WO 95/30682 and WO 96/04293) and JP-A No.10-245390.

In European Patent Publication No.0528030A1, there is a description of the compounds relating to the present invention, wherein the group corresponding to -NH(CH₂)ₘ-R of the present invention is H exclusively. US Patent 5,591,842 discloses the compounds in which -(CH₂)ₘ-R and H attached to the exocyclic nitrogen atom in the compounds of the present invention are represented by R¹ and R² including a wide range of groups; in this patent, however, the closest compounds to the present invention are those in which R¹ and R² each is furyl, thienyl or pyridyl (one of R¹ and R² may possibly be H). US Patent 5,804,564 describes the compounds in which the group corresponding to -(CH₂)ₘ-R in the compounds of the present invention is bis(hydroxymethyl)methyl exclusively. WO 96/04293 describes the compounds in which the group corresponding to -NH(CH₂)ₘ-R in the compounds of the present invention is R¹, which has a wide range of groups. In this case, however, no compound closely related to the present invention is disclosed and moreover the group corresponding to G in the compounds of the present invention is defined as a disaccharide group.

JP-A No.10-245390 discloses the compounds of the following formula: 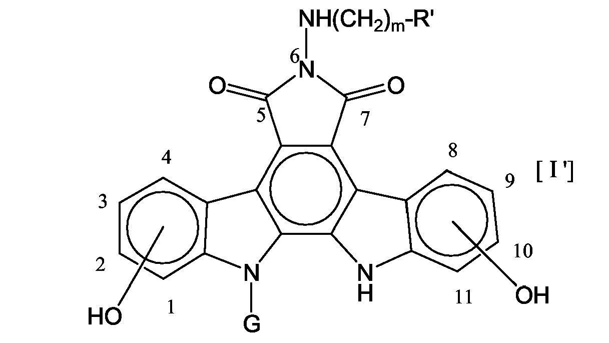 wherein R' represents a phenyl, naphthyl, pyridyl, furyl or thienyl group which has one or two substituents selected from the group consisting of hydroxy, lower alkoxy group, hydroxy lower alkyl group and hydroxy lower alkenyl group, with the proviso that when R' has a lower alkoxy group as a substituent, R' has an additional group selected from the group consisting of hydroxy, lower alkoxy group, hydroxy lower alkyl group and hydroxy lower alkenyl group; m represents an integer of 1 to 3; G represents a β-D-glucopyranosyl group; and the hydroxyl group on the indolopyrrolocarbazole ring is located at the 1-and 11-posiitons or 2- and 10-positions, or a pharmaceutically acceptable salt thereof. In the description of JP-A No.10-245390, however, there is no disclosure or suggestion that R' in the compounds of the formula [I'] is a pyridyl, furyl or thienyl group with no substituent, though R' is defined as a phenyl, naphthyl, pyridyl, furyl or theinyl group having one or two substituents.

### Disclosure of Invention

The present inventors have found that among the compounds of the formula [I'] those in which R' is an unsubstituted pyridyl, furyl or thienyl group have much more potent anti-tumor effect than those in which R' is a substituted pyridyl, furyl or thienyl group.

Thus the present invention relates to a compound represented by the formula: 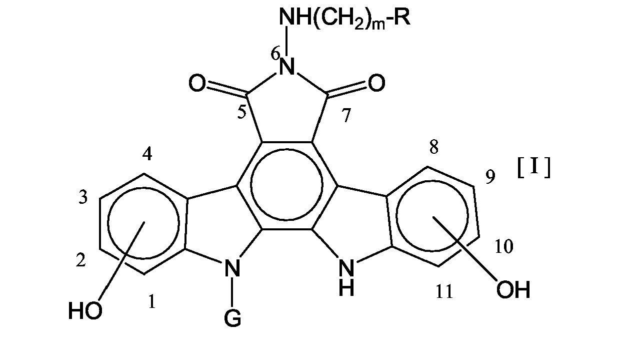 wherein R represents an unsubstituted pyridyl, furyl, or theinyl group; m represents an integer of 1 to 3; G represents a β-D-glucopyranosyl group; and the hydroxyl group on the indolopyrrolocarbazole ring is located at the 1- and 11-positions or 2- and 10-positions, or a pharmaceutically acceptable salt thereof, and the use thereof as an anti-tumor agent.

Also, the present invention relates to an anti-tumor agent which comprises an effective amount of the compound of the formula [I] or a pharmaceutically acceptable salt thereof sufficient to exhibit anti-tumor activity and a pharmaceutically acceptable carrier or diluent.

### Best Mode for Carrying Out the present invention

In the definition of the substituents in the compounds of the present invention represented by the formula [I], as for R, an unsubstituted pyridyl group, particularly pyridin-4-yl group is preferred. m indicates an integer of 1 to 3, with 1 being preferred.

The hydroxyl group on the indolopyrrolocarbazole ring may be located at either 1- and 11-positions or 2- and 10-positions, preferably at the 2- and 10-positions.

The compound of the present invention includes, preferably, compounds represented by the formula: 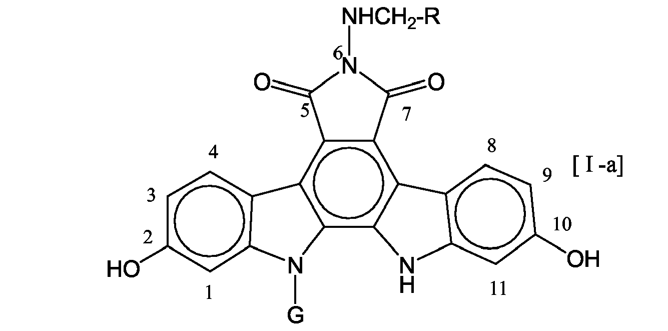 wherein R and G each has the same meanings as mentioned above,
or a pharmaceutically acceptable salt thereof; or a compound represented by the formula: 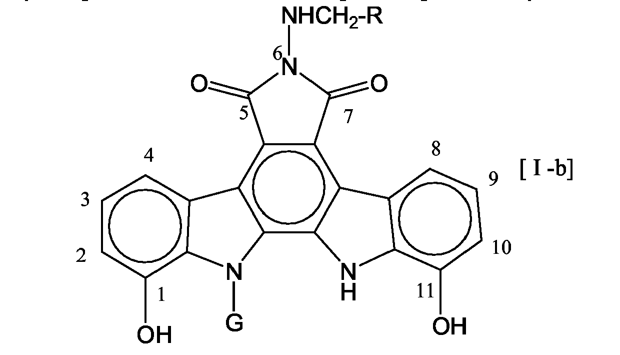 wherein R and G each has the same meanings as mentioned above, or a pharmaceutically acceptable salt thereof.

The followings will explain the process for producing the compounds of the present invention.

The indolopyrrolocarbazole derivatives of the present invention can be produced from publicly known compounds described in European Patent Publication Nos. 0528030A1 and 0545195A1, WO 95/30682 and WO 96/04293 by reacting a compound of the formula: 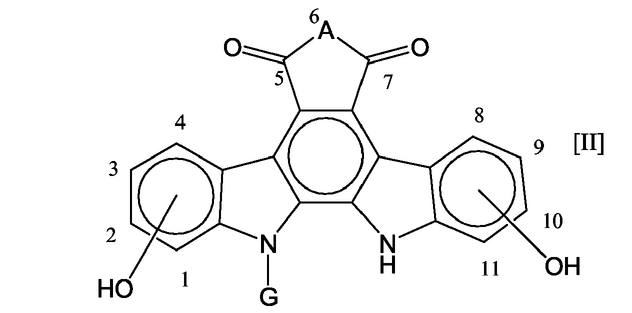 wherein A represents NH; and G has the same meanings as mentioned above, with a compound of the formula:

H₂N-NH(CH₂)ₘR [III]

wherein R and m each has the same meanings as mentioned above,
or alternatively by condensing a compound of the formula: 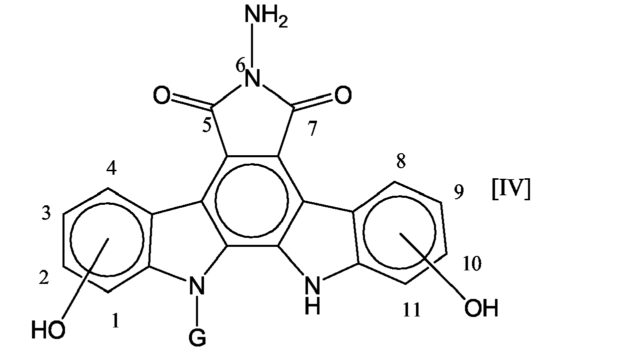 wherein G has the same meanings as mentioned above,
with a compound of the formula:

R¹(CH₂)ₘCHO [V]

wherein R' has the same meanings as R; and m has the same meanings as mentioned above, followed by reduction and deprotection if necessary, or by reacting a compound of the formula [IV] with a compound of the formula:

R₁(CH₂)ₘL [VI]

wherein L represents a leaving group; and R₁ and m each has the same meanings as mentioned above, followed by deprotection if necessary.

The reaction of a compound of the formula [II] with a compound of the formula [III] is based on the chemically well known reaction of an imide or acid anhydride with a hydrazine derivative. In carrying out the reaction, a solvent which has no adverse effect on the usual reaction such as, for example, tetrahydrofuran and N,N-dimethylformamide can be used. The compound [III] may be used usually in slightly excessive amount over that of the compound [II] up to 5 molar equivalent to the compound [II], if necessary in a large excess.

The reaction may be carried out usually at a temperature of from -50°C to the boiling point of the solvent, if required at a lower temperature or higher temperature than the defined range. The reaction may be conducted usually for a period of 30 minutes to 2 days, if required for a shorter or longer period than the time as determined.

The condensation reaction of a compound of the formula [IV] with a compound of the formula [V] and the subsequent reduction to give the compound [I] can be carried out in the same reaction system, though a Schiff base (hydrazone) as an intermediate may be once isolated if required. That is, in general, the compound [IV] is mixed with the compound [V] in a proper solvent, to which a reducing agent is added. In such a case, it is appropriate to carry out the reaction in the presence of an acid such as acetic acid or hydrochloric acid. The solvent used in this reaction includes, for example, alcohol solvents such as methanol or ethanol, or aprotic polar solvents such as N,N-dimethylformamide. The reduction of a Schiff base (hydrazone) may be achieved by a metal hydride complex such as sodium cyanoborohydride, or by catalytic hydrogenation.

The reaction of a compound of the formula [IV] with a compound of the formula [VI] is based on the alkylation reaction of an amine, which may be achieved, for example, by reaction with an alkyl halide, alkyl mesylate or alkyl tosylate.

The product produced in the above reaction may be purified by a publicly known method used in the organic chemistry field, such as precipitation, extraction with an organic solvent, recrystallization, chromatography, etc.

Moreover, the present invention includes the pharmaceutically acceptable salts of the compounds as produced in the above process. Such salts are exemplified by salts with an alkali metal such as potassium or sodium, salts with an alkaline earth metal such as calcium, salts with a basic organic compound such as ethylamine or arginine, salts with an inorganic acid such as hydrochloric acid or sulfuric acid, or salts with an organic acid such as acetic acid, citric acid or maleic acid.

The followings will illustrate the results of the drug effect tests made for the compounds of the formula [I] in the present invention.

### Method for determining the effects of the agent on cells

The compounds of the formula [I] of the present invention exhibit potent cell-growth inhibitory effects against the tumor cells (MKN-45) of human origin as shown in Table 1.
a) Reagents
   Fetal calf serum (FCS) was obtained from Molgate and the DMEM culture medium from Asahi Technoglass Inc.
b) Cells
   Human gastric cancer cell MKN-45 was obtained from the IBL Co., Ltd.
c) Method for determining the effect
   The cells were suspended in 10% FCS-DMEM medium and the suspension was distributed in a 96-well plastic plate in the amount of 1000 cells/50 microliter/well. The plate was incubated at 37°C in 5% CO₂-95% air overnight. Each drug was dissolved in dimethylsulfoxide or a suitable solvent by serial dilution and distributed in the wells at 50 microliters per well in which the cells had been added. The plate was incubated at 37°C in 5% CO₂-95% air for 3 days. The growth of the cultured cells was measured by the WST-8 method (H. Tominaga et al., Anal. Commun., 36, 47-50 (1999)). In the WST-8 method, 10 microliter of WST-8 reagent solution was added, and then incubated at 37°C for 1 to 6 hours. After stirring the plate, the resulting formazan was quantitatively analyzed by colorimetry to determine the inhibition rate of the drug. Thus, 50% inhibitory concentration (IC50) for the growth was determined for each compound.

**Table 1 Cell-growth inhibitory activities of the agent against MKN-45(human stomach cancer cells)**

| No. of examples | 50% tumor growth inhibiting activity (IC50, µM) |
|---|---|
| 1 | 0.00071 |
| 2 | 0.0014 |
| 3 | 0.00095 |
| 4 | 0.0063 |
| 5 | 0.0028 |
| 6 | 0.0050 |
| 7 | 0.0027 |
| 8 | 0.0041 |

### Method for determining the effects of the agent on animals

a) Mice and cancer cells
Female nude mice of 5 weeks of age (Balb/c-nu/nu) were obtained from Clea Japan, Inc. Human lung cancer cells LX-1 were obtained from the Japanese Foundation for Cancer Research Institute.
b) Reagents
The synthesis of the control compound is described in JP-A No.10-245390, Example 14.
c) Method for determining the effect
The cancer cells were transplanted subcutaneously to nude mice so that solid cancer can be maintained. The solid cancer was sliced into 3 mm cubic blocks in physiological saline under ice cooling. One of the blocks was transplanted subcutaneously to a nude mouse to be used in experiment (Day 1). The cancer cells were grown subcutaneously in the mouse to form a tumor mass. The size of the mass was measured with a lapse of time, and when the volume reached 0.2 cm³ or more (approximation by volume= major axis x minor axis² ÷ 2), administration of the drug was started. The drug was dissolved in 5% glucose solution and administered intravenously at the tail successively twice a week for 2 weeks (total 4 times). Two weeks after the administration, the percentage (T/C%) of the tumor volume (T) was calculated from the tumor volume (C) in the control group and that (T) in the test group as a tumor volume ratio.
d) Results

**Table 2**

| Ratio of tumor volume (Dose : mg/m², in total dose) | | |
|---|---|---|
| Treated/Control (%) | | |
| | Compound of Example 1 | Control Compound |
| LX-1 | 1% (1200 mg/m²) | 11% (1000 mg/m²) |

As seen from Table 2, the compound of Example 1 exhibited markedly a stronger anti-tumor activity against human lung cancer cells than the control compound. In other words, in comparison with the control compound in which R is a substituted pyridyl group, the compound (in Example 1) in which R is an unsubstituted pyridyl group is recognized to have a much more potent activity.

Accordingly, the pharmaceutical compositions or anti-tumor agents containing as an active ingredient a compound of the formula [I] in the present invention together with a pharmaceutically acceptable carrier or diluent are expected to be promising.

Also, the compounds of the present invention, since they have a potent anti-tumor activity, are useful as anti-tumor agents for humans or other mammals, particularly for humans, in prevention or treatment of solid tumors such as head and neck cancer, thyroid cancer, lung cancer, esophageal cancer, stomach cancer, hepatic cancer, pancreatic cancer, colon cancer, renal carcinoma, prostatic cancer, testis cancer, uterus cancer, ovarian cancer, breast cancer, brain tumor, and so on, and other cancers such as leukemia, lymphoma, myeloma, and so on, and preferably in prevention or treatment of stomach cancer, colon cancer, lung cancer and breast cancer, particularly for lung cancer.

The compound of the present invention can be used in an anti-tumor pharmaceutical preparations appropriate for oral or parenteral administration, which are obtained by admixing it with a conventional solid or liquid diluent or carrier publicly known in the field of pharmaceutical technology. The oral administration preparation includes, for example, tablets, capsules, powder, granules, liquid preparation, and the like. The parenteral administration preparation includes, for example, sterilized liquid preparation such as solutions or suspensions.

The solid preparation may be prepared as tablets, capsules, granules or powder, if necessary together with a suitable additive. Such additive includes, for example, saccharides such as lactose or glucose; starches such as corn, wheat or rice; fatty acids such as stearic acid; inorganic salts such as magnesium metasilicate aluminate or anhydrous calcium phosphate; polymers such as polyvinylpyrrolidone or polyalkylene glycol; fatty acid salts such as calcium stearate or magnesium stearate; alcohols such as stearyl alcohol or benzyl alcohol; synthetic cellulose derivatives such as methylcellulose, carboxymethylcellulose, ethylcellulose or hydroxypropylmethylcellulose; and other additives such as water, gelatin, talc, vegetable oil, gum arabic, and the like.

The solid preparations such as tablets, capsules, granules or powder generally contain 0.1-100% by weight, preferably 5-100% by weight of the active ingredient.

The liquid preparations may be prepared in the form of suspension, syrup or injection using a suitable additive usually used in liquid preparations, for example, water, alcohol, or plant-derived oil such as soybean oil, peanut oil or sesame oil.

Particularly, when the liquid preparation is parenterally administered as intramuscular injection, intravenous injection or subcutaneous injection, there can be mentioned a suitable solvent such as distilled water for injection, aqueous solution of lidocaine hydrochloride (for intramuscular injection), physiological saline, aqueous solution of glucose, ethanol, polyethylene glycol, liquid for intravenous injection (e.g., aqueous solution of citric acid and sodium citrate) or electrolyte solution (for intravenous infusion or injection), or mixtures thereof.

The preparations for injection may be in a form of solutions prepared in advance or in intact powder or a mixture with a suitable additive which can readily be dissolved at the time of use. The preparations for injection generally contain 0.1-10% by weight, preferably 1-5% by weight of the active ingredient.

The oral liquid preparations such as suspensions or syrup contain 0.5-10% by weight of the active ingredient.

It should be noted that the practically preferred dose of the compound of the present invention depends on the kind of the compound used, the kind of the composition prepared, administration frequency and the site to be treated, and the host and tumor. For example, the daily dose for an adult is in the range of from 10 to 500 mg in the case of oral administration and in the range of from 10 to 100 mg in the case of parenteral administration, preferably in intravenous injection. The frequency of the administration is 1 to 5 times depending on the route of administration and the diseases. The administration may be made intermittently, for example every other day or at intervals of two days.

### Examples

The present invention will be further explained by the following examples; however, the present invention is not limited to these examples.

In the examples, the compounds represented by the following structural formula, used as a starting material of working examples, are hereinafter referred to as Compound A. The manufacturing method of Compound A has been disclosed in Example 47 of the Japanese Patent No.2629542. 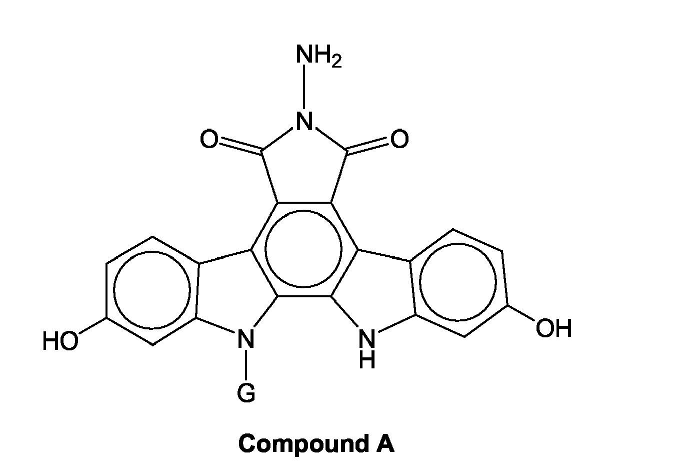 wherein G represents a β-D-glucopyranosyl group.

### Example 1

Synthesis of the compound of the formula:

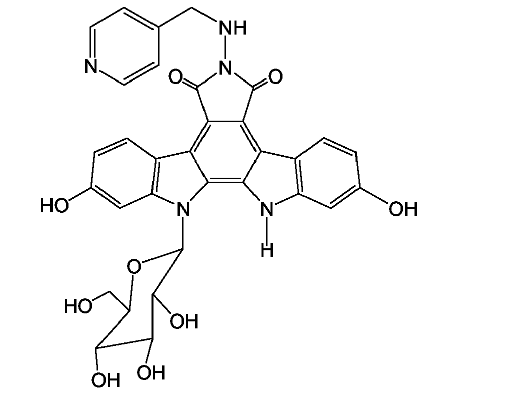

To a solution of 1.0 g of Compound A and 253 mg of 4-pyridinecarbaldehyde dissolved in 200 ml of methanol, 0.3 ml of acetic acid was added, and the mixture was stirred at 80°C overnight to give a crystal as a precipitate, which was collected by filtration and washed with chloroform. The crystals were dissolved in methanol/tetrahydrofuran (1:1); 10% palladium-carbon was then added thereto; and the mixture was stirred in hydrogen atmosphere overnight. The mixture was then filtered through celite and condensed, and the residue was chromatographed on a column of Sephadex LH-20 and was eluted with methanol. The fraction containing the objective compound was evaporated to dryness to give 730 mg of the title compound.
Rf: 0.12 (Merck; Kiesel gel 60F₂₅₄; developing solvent: toluene/acetonitrile/tetrahydrofuran/water/acetic acid = 2 : 4 : 2 : 0.5 : 0.1)
FAB-MS (m/z): 626 (M+H)⁺
¹H-NMR (300MHz, DMSO-d₆, δppm): 11.18 (1H, s), 9.80 (2H, br), 8.89 (1H, d, J=8.3Hz), 8.76 (1H, d, J=8.6Hz), 8.49 (2H, dd, J=1.8, 5.7Hz), 7.55 (2H, d, J=6.0Hz), 7.16 (1H, d, J=1.5Hz), 6.97 (1H, d, J=2.4Hz), 6.81 (2H, dt, J=2.4, 8.6Hz), 6.32 (1H, t, J=4.8Hz), 5.96 (1H, d, J=9.0Hz), 5.85 (1H, br), 5.34 (1H, br), 5.14 (1H, br), 4.90 (1H, br), 4.34 (2H, d, J=4.2Hz), 4.00 (1H, d, J=11.1Hz), 3.90 (2H, br), 3.73-3.80 (1H, m), 3.50 (2H, br).

### Example 2

Synthesis of the compound of the formula:

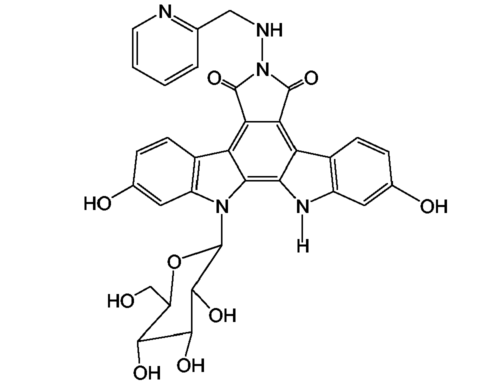

To a solution of Compound A (45 mg) and 5 equivalents of 2-pyridinecarboxyaldehyde dissolved in methanol (9 ml), 60 µl of acetic acid was added, and the mixture was stirred at 80°C overnight. The mixture was condensed, and the precipitated solid was washed with chloroform-hexane and dried. The resulting solid (20 mg) was dissolved in 5 ml of tetrahydrofuran; a tetrahydrofuran solution (5 ml) containing sodium cyanoborohydride (NaBH₃CN)(80 mg) and 1M zinc chloride tetrahydrofuran solution (0.64 ml) were added thereto; and the mixture was stirred overnight. The reaction mixture was condensed, and the residue was purified by chromatography on Dia-Ion HP-20 column (methanol) and then on Sephadex LH-20 column (methanol) to give the title compound (10.5 mg).
Rf: 0.07 (Merck; Kiesel gel 60F₂₅₄; developing solvent: acetonitrile/tetrahydrofuran/toluene/water/acetic acid = 4 : 2 : 2 : 0.5 : 0.1)
FAB (m/z): 626 (M+H)⁺
¹H-NMR (300MHz, DMSO-d₆, δppm): 11.19 (1H, s), 9.78 (1H, s), 9.75 (1H, s), 8.85 (1H, d, J=8.5Hz), 8.76 (1H, d, J=8.6Hz), 8.41 (1H, d, J=4.4Hz), 7.79 (2H, m), 7.23 (1H, m), 7.17 (1H, d, J=2.1Hz), 6.98 (1H, d, J=2.1Hz), 6.78-6.84 (2H, dt, J=2.1Hz, 8.6Hz), 6.25 (1H, t, J=4.5Hz), 5.96 (1H, d, J=8.1Hz), 5.86 (1H, t, J=4.2Hz), 5.33 (1H, d, J=4.2Hz), 5.12 (1H, d, J=5.1Hz), 4.91 (1H, d, J=5.1Hz), 4.36 (2H, d, J=4.8Hz), 3.90-4.02 (3H, m), 3.75-3.80 (1H, m), 3.50 (2H, m).

### Example 3

Synthesis of the compound of the formula:

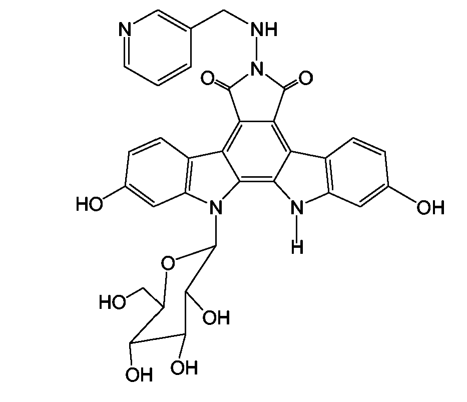

To a solution of 50 mg of Compound A and 50 mg of 3-pyridinecarbaldehyde dissolved in 10 ml of methanol, 50 µl of acetic acid was added, and the mixture was stirred at 80°C for 6 hours. Then, a chloroform-normal hexane mixture solvent was added to the reaction mixture to give 47.3 mg of the hydrazone derivative as precipitate which was collected by filtration. The resulting hydrazone derivative (25 mg) was dissolved in methanol/ tetrahydrofuran (1:1); 20 mg of 10% palladium-carbon was then added thereto; and the mixture was stirred in hydrogen atmosphere overnight. The mixture was then filtered through celite and condensed to give a residue, and then 1 ml of 10% HCl-MeOH was added thereto and was condensed. Water and ethyl acetate were added to the residue, and the aqueous layer was extracted and treated with triethylamine to neutralize the hydrochloride. The aqueous solution was chromatographed and developed on an HP-20 column and eluted with methanol. The eluate was condensed, and the residue was chromatographed on a column of Sephadex LH-20 and eluted with methanol. The fraction containing the objective compound was evaporated to dryness to give 3.5 mg of the title compound.
FAB-MS (m/z): 626 (M+H)⁺
¹H-NMR (300MHz, DMSO-d₆, δppm): 11.16 (1H, s), 9.85 (2H, br), 8.83 (1H, d, J=8.5Hz), 8.75 (1H, d, J=8.6Hz), 8.62 (1H, d, J=2.4Hz), 8.41 (1H, dd, J=1.8, 5.1Hz), 7.92 (1H, dt, J=2.4, 8.1Hz), 7.33 (1H, dd, J=5.1, 8.1Hz), 7.15 (1H, s), 6.96 (1H, d, J=1.5Hz), 6.80 (2H, dt, J=1.5, 6.3Hz), 6.26 (1H, t, J=4.2Hz), 5.95 (1H, d, J=7.8Hz), 5.88 (1H, br), 5.38 (1H, br), 5.16 (1H, br), 4.92 (1H, br), 4.29 (2H, d, J=4.5Hz), 4.02 (1H, d, J=10.8Hz), 3.90 (2H, m), 3.76 (1H, d, J=10.5Hz), 3.50 (2H, m).

### Example 4

Synthesis of the compound of the formula:

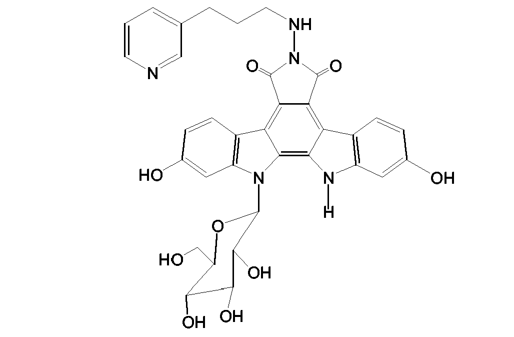

To a solution of Compound A (50 mg) and 3-(3-pyridyl)propanal (183 mg) dissolved in methanol (10 ml), 10 µl of acetic acid was added, and the mixture was stirred at 80°C for 3 hours. The reaction mixture was condensed, and the residue was solidified with ethyl acetate. The resulting solid (5.3 mg) was dissolved in 1 ml of tetrahydrofuran; NaBH₃CN (10 mg) and 10% HC1-MeOH (1 ml) were added thereto; and the mixture was stirred at room temperature for 3 hours. The reaction mixture was condensed, and the residue was chromatographed on a Sephadex LH-20 column and eluted with methanol. The fraction containing the objective compound was evaporated to dryness to give 1.3 mg of the title compound.
Rf: 0.10 (Merck; Kiesel gel 60F₂₅₄; developing solvent: acetonitrile/tetrahydrofuran/toluene/water/acetic acid= 4 : 2: 2: 0.5 : 0.1)
FAB (m/z): 654 (M+H)⁺
¹H-NMR (300MHz, DMSO-d₆, δppm): 1.72-1.82 (2H, m), 2.80 (2H, t, J=7.3Hz), 3.00-3.06 (2H, m), 3.48-3.53 (2H, m), 3.75-4.04 (4H, m), 4.93 (1H, br), 5.18 (1H, bs), 5.41 (1H, bs), 5.79 (1H, t, J=4.2Hz), 5.92 (2H, s), 5.96 (1H, d, J=8.3Hz), 6.78-6.83 (2H, m), 6.98 (1H, d, J=2.0Hz), 7.16 (1H, s), 7.28 (1H, dd, J=7.5Hz, 4.5Hz), 7.67 (1H, dt, J=7.5Hz, 2.0Hz), 8.36 (1H, dd, J=4.5Hz, 2.0Hz), 8.47 (1H, s), 8.78 (1H, d, J=8.5Hz), 8.87 (1H, d, J=8.5Hz), 9.83 (1H, br, 11.18 (1H, s).

### Example 5

Synthesis of the compound of the formula:

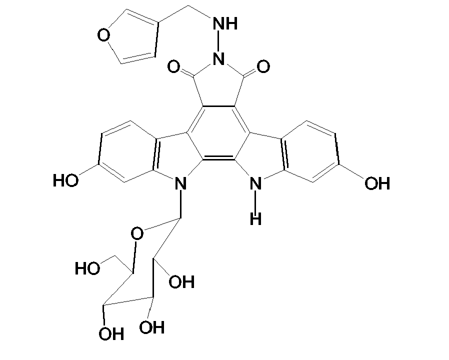

To a solution of Compound A (200 mg) and 3-furancarboxyaldehyde (100 mg) dissolved in methanol (10 ml), 18 µl of acetic acid was added, and the mixture was stirred at 80°C for 1 hours. The reaction mixture was condensed, and the residue was solidified with ethyl acetate. The resulting solid (30 mg) was dissolved in tetrahydrofuran (2 ml); NaBH₃CN (20 mg) and 10% HC1-MeOH (2 ml) were added thereto; and the mixture was stirred at room temperature for 1 hour. The reaction mixture was condensed, and the residue was chromatographed on a Sephadex LH-20 column and eluted with methanol. The fraction containing the objective compound was evaporated to dryness to give the title compound (23.2 mg).
Rf: 0.60 (Merck; Kiesel gel 60F₂₅₄; developing solvent: acetonitrile/tetrahydrofuran/toluene/water/acetic acid= 4 : 2: 2: 0.5 : 0.1)
FAB (m/z): 615 (M+H)⁺
¹H-NMR (300MHz, DMSO-d₆, δppm): 11.18 (1H, s), 9.77 (2H, br), 8.85 (1H, d, J=8.6Hz), 8.77 (1H, d, J=8.5Hz), 7.62 (1H, s), 7.50 (1H, d, J=1.0Hz), 7.16 (1H, s), 6.97 (1H, d, J=1.9Hz), 6.78-6.83 (2H, m), 6.57 (1H, s), 5.94-5.99 (2H, m), 5.87 (1H, br), 5.34 (1H, br), 5.12 (1H, br), 4.91 (1H, br), 4.11 (2H, d, J=4.6Hz), 3.75-4.06 (4H, m), 3.47-3.53 (2H, m).

### Example 6

Synthesis of the compound of the formula:

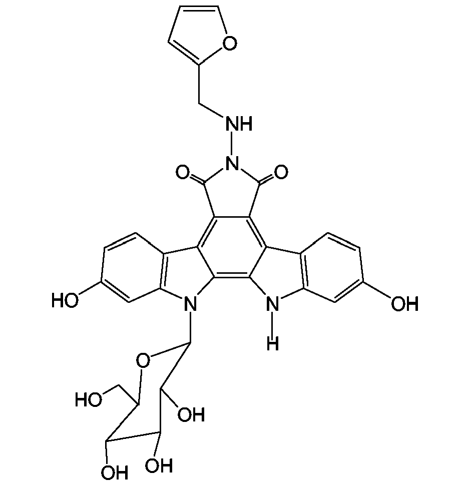

To a solution of Compound A (200 mg) and 2-furancarboxyaldehyde (100 mg) dissolved in methanol (10 ml), 18 µl of acetic acid was added, and the mixture was stirred at 80°C for 6 hours. The reaction mixture was condensed, and the residue was solidified with ethyl acetate. The resulting solid (20 mg) was dissolved in methanol-tetrahydrofuran (1:1)(5 ml); NaBH₃CN (20 mg) and 10% HCl-MeOH (2 ml) were added thereto; and the mixture was stirred at room temperature for 0.5 hours. The reaction mixture was condensed, and the residue was chromatographed on a Sephadex LH-20 column and eluted with methanol. The fraction containing the objective compound was evaporated to dryness to give the title compound (10.8 mg).
Rf: 0.30 (Merck; Kiesel gel 60F₂₅₄; developing solvent: acetonitrile/tetrahydrofuran/toluene/water/acetic acid= 4 : 2: 2: 0.5 : 0.1)
FAB (m/z): 615 (M+H)⁺
¹H-NMR (300MHz, DMSO-d₆, δppm): 11.10 (1H, s), 9.50-10.20 (2H, br), 8.82 (1H, d, J=8.5Hz), 8.73 (1H, d, J=8.5Hz), 7.55 (1H, s), 7.13 (1H, s), 6.95 (1H, d, J=1.9Hz), 6.74-6.78 (2H, m), 6.31-6.35 (2H, m), 6.05 (1H, t, J=5.1Hz), 5.94 (1H, d, J=8.3Hz), 5.75-6.00 (1H, br), 4.83-5.50 (3H, m), 4.12 (2H, d, J=4.6Hz), 3.73-4.05 (4H, m), 3.49-3.53 (2H, m).

### Example 7

Synthesis of the compound of the formula:

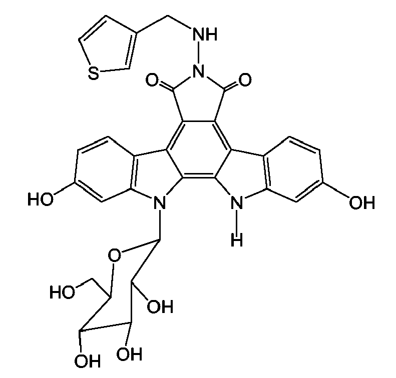

To a solution of 30 mg of Compound A (hydrazine derivative) and 30 mg of 3-thiophenecarboxyaldehyde dissolved in 6 ml of methanol, 30 µl of acetic acid was added, and the mixture was stirred at 80°C for 2 hours. The reaction mixture was cooled to room temperature; an appropriate amount of NaBH₃CN and 10% HCl-MeOH were added thereto; and the mixture was stirred at room temperature for 30 minutes. An appropriate amount of water was added, and the mixture was extracted with a mixture solvent of ethyl acetate and methyl ethyl ketone. The organic layer was condensed, and the residue was chromatographed on a Sephadex LH-20 column and eluted with methanol. The fraction containing the objective compound was evaporated to dryness to give the title compound (5.5 mg) as a red solid.
FAB (m/z): 631 (M+H)⁺
¹H-NMR (300MHz, DMSO-d₆, δppm): 3.50 (2H, m), 3.78 (1H, m), 3.91 (2H, s), 4.02 (1H, m), 4.27 (2H, d, J=4.5Hz), 4.91 (1H, d, J=5.1Hz), 5.12 (1H, d, J=3.9Hz), 5.33 (1H, d, J=3.6Hz), 5.87 (1H, t, J=3.6Hz), 5.96 (1H, d, J=8.4Hz), 6.06 (1H, t, J=4.5Hz), 6.80 (1H, dd, J=2.4Hz, 8.4Hz), 6.82 (1H, dd, J=2.1Hz, 8.7Hz), 6.98 (1H, d, J=2.4Hz), 7.17 (1H, d, J=2.1Hz), 7.22 (1H, dd, J=2.4Hz, 3.6Hz), 7.45 (1H, s), 7.47 (1H, t, J=2.4Hz), 8.78 (1H, d, J=8.7Hz), 8.86 (1H, d, J=8.4Hz), 9.76 (1H, s), 11.18 (1H, s).

### Example 8

Synthesis of the compound of the formula:

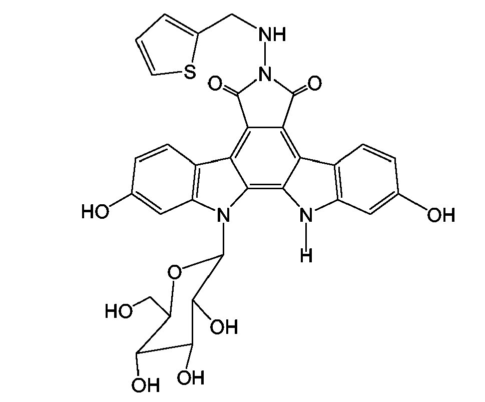

100 mg of Compound A (hydrazine derivative) and 50 mg of 2-thiophenecarboxyaldehyde was dissolved in 3 ml of DMF, and was stirred at 80°C overnight. An appropriate amount of water was added, and the mixture was extracted with ethyl acetate. The organic layer was condensed, and the residue was chromatographed on a Sephadex LH-20 column and eluted with methanol. The fraction containing the reaction product (hydrazone) was condensed to give 88 mg of the hydrazone. The resulting hydrazone (40 mg) was dissolved in 5 ml of methanol; 8 mg of NaBH₃CN and a proper amount of 10% HCl-MeOH were added thereto; and the mixture was stirred at room temperature for 2 hours. The reaction mixture was condensed, and the residue was chromatographed on a Sephadex LH-20 column and eluted with methanol. The fraction containing the objective compound was evaporated to dryness to give the title compound (35 mg) as an orange solid.
FAB (m/z): 631 (M+H)⁺
¹H-NMR (300MHz, DMSO-d₆, δppm): 3.45-3.56 (3H, m), 3.77 (1H, m), 3.85-4.12 (2H, m), 4.44 (2H, d, J=4.8Hz), 4.92 (1H, d, J=4.8Hz), 5.11 (1H, d, J=4.2Hz), 5.32 (1H, d, J=4.5Hz), 5.85 (1H, t, J=3.6Hz), 5.97 (1H, d, J=8.4Hz), 6.16 (1H, t, J=4.8Hz), 6.81 (1H, t, J=8.7Hz), 6.90 (1H, m), 6.97 (1H, s), 7.06 (1H, d, J=3.0Hz), 7.18 (1H, s), 7.45 (1H, s), 7.40 (1H, t, J=4.8Hz), 8.77 (1H, d, J=8.4Hz), 8.85 (1H, d, J=9.3Hz), 9.75 (1H, s), 9.78 (1H, s), 11.19 (1H, s).

### Industrial Applicability

The compounds of the present invention have a potent anti-tumor effect and are useful as anti-tumor agents in the medicinal field.

## Claims

1. A compound represented by the following formula: 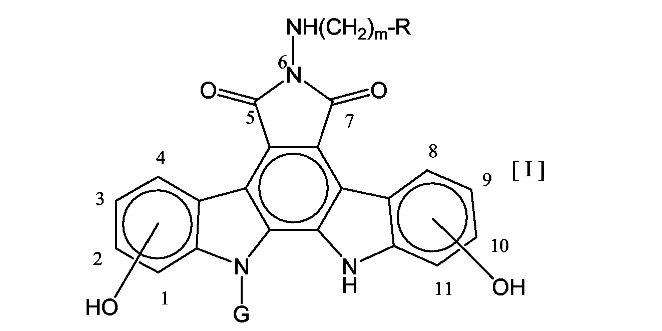 wherein R represents an unsubstitued pyridyl, furyl, or thienyl group,
m represents an integer of 1 to 3,
G represents a β-D-glucopyranosyl group, and the positions of substitution of the hydroxyl groups on the indolopyrrolocarbazole ring are the 1- and 11-positions, or the 2- and 10-positions,
or a pharmaceutically acceptable salt thereof.

2. The compound according to Claim 1, represented by the following formula: 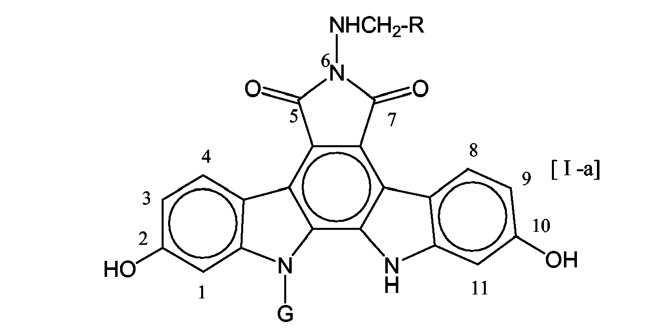 wherein R and G are the same as defined in Claim 1,
or a pharmaceutically acceptable salt thereof.

3. The compound according to Claim 2, wherein R is a pyridine-4-yl group, or a pharmaceutically acceptable salt thereof.

4. The compound according to Claim 1, represented by the following formula: 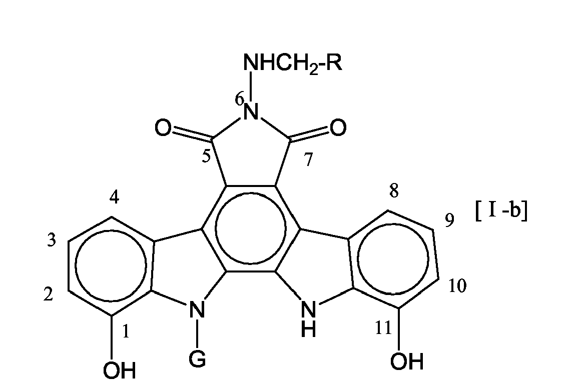 wherein R and G are the same as defined in Claim 1,
or a pharmaceutically acceptable salt thereof.

5. An anti-tumor agent comprising, together with a pharmaceutically acceptable carrier or diluent, an effective amount of the compound according to Claim 1 or a pharmaceutically acceptable salt thereof, sufficient to exhibit antitumor activity

6. The anti-tumor agent according to Claim 5 which is used for the treatment of lung cancer.
